Europäisches Patentamt

European Patent Office

Office européen des brevets

(19)

(11) Publication number: **0 161 328 B1**

# EUROPEAN PATENT SPECIFICATION

(12)

(45) Date of publication of patent specification: 05.06.91

(51) Int. Cl.⁵ **G01N 33/569**, G01N 33/546, C12N 7/06, C12N 7/02

(21) Application number: 84105636.9

(22) Date of filing: 17.05.84

The file contains technical information submitted after the application was filed and not included in this specification

(54) Supported viral antigen and preparation and use thereof.

(43) Date of publication of application:
21.11.85 Bulletin 85/47

(45) Publication of the grant of the patent:
05.06.91 Bulletin 91/23

(84) Designated Contracting States:
BE DE FR GB IT NL SE

(56) References cited:
EP-A- 0 001 838
EP-A- 0 054 249
GB-A- 2 001 326
US-A- 3 622 663
US-A- 4 195 074

CHEMICAL ABSTRACTS, volume 78, no. 3, January 22, 1973, page 256, abstract 14339q, (COLUMBUS, OHIO, US); A.A. SALMI: "Characterization of a structural antigen of rubella virus reacting by gel precipitation", & Acta Pathol. Microbiol. Scand., Sect. B 1972, 80(4), 534-44

CHEMICAL ABSTRACTS, volume 99, no. 1, July 4, 1983, page 275, abstract 2755t, (COLUMBUS, OHIO, US); M.N. WAXHAM et al.:

"Immunochemical identification of rubella virus hemagglutinin", & Virology 1983, 126(1), 194-203

CHEMICAL ABSTRACTS, volume 70, no. 1, January 6, 1969, page 98, abstract 1147k, (COLUMBUS, OHIO, US); A.E. AULETTA et al.: "Effect of sodium deoxycholate on rubella virus", & Appl. Microbiol. 1968, 16(10), 1624

(73) Proprietor: Dorsett, Preston H.
5415 Longwood Avenue
Memphis Tennessee 38134(US)

(72) Inventor: Dorsett, Preston H.
5415 Longwood Avenue
Memphis Tennessee 38134(US)

(74) Representative: von Kreisler, Alek, Dipl.-Chem. et al
Deichmannhaus am Hauptbahnhof
W-5000 Köln 1(DE)

EP 0 161 328 B1

## Description

This invention relates to the production of virus antigens for the production of sensitized solids. Particularly, the invention relates to a process for producing a solid support sensitized with soluble rubella virus antigen as defined below.

United States Patent No. 4,195,074 discloses a process for producing soluble rubella virus antigen, and the use thereof in an agglutination test for rubella virus antibody. In accordance with U.S. Patent 4,195,074, the tissue culture from rubella virus infected cells is subjected to immunosorbent separation through a column containing IgG derived from human serum known to contain antibodies reactive with rubella antigen followed by elution of the rubella antigen material from the column and selection of the soluble antigen by gel permeation chromatography. The antigen may then be employed for sensitizing erythrocytes, and the sensitized erythrocytes are used to determine antibody in human serum samples by direct agglutination.

In accordance with the aforesaid patent, the so-called rubella antigen is not recovered from the virus, per se, and, therefore, it is believed that such material does not include structural proteins of the virus.

In EP-A-54249 there are described immumoparticles and a process for their preparation. According to a particular embodiment the disruption of the micro-organism is carried out by ultrasonic wave. An appropriate disruption of the specific micro-organism rubella virus is not described herein.

The present invention provides a process for producing a solid support sensitized with soluble rubella virus antigen comprising:

deriving soluble rubella virus antigen having at least one structural protein of the virus and being immunoreactive with early phase rubella virus antibody by disruption of whole rubella virus with a surfactant or detergent for a time and in an amount sufficient to disrupt the virus without destroying its antigenic characteristics and

supporting the solubilized antigen on a solid support.

Soluble rubella virus antigen is obtained from whole rubella virus.

In accordance with a further aspect of the present invention, there is provided a process for producing purified virus by the use of an adsorption gel to remove non-viral proteins and nucleic acids.

In accordance with yet a further aspect of the invention, there is provided a method for producing a solid support sensitized with a viral antigen.

More particularly, the rubella virus antigen is isolated from intact rubella virus by treating purified whole rubella virus with a surfactant or detergent which disrupts the virus to provide the soluble rubella virus antigen, without destroying the antigenic characteristics thereof. The detergent is employed in an amount that is sufficient to disrupt and solubilize the whole virus without destroying its antigenic characteristics.

The surfactant or detergent which is used for disrupting the whole rubella virus may be any one of a wide variety of surfactants or detergents which disrupt and solubilize the virus, without destroying the antigenic characteristics, including cationic, anionic and non-ionic surfactants. Such surfactants are well known in the art, and as representative examples, there may be mentioned alkali metal salts of sulfates, soaps, sulfated or sulfonated oils, various amines, quaternary salts and condensation products with ethylene oxide. Such detergents and surfactants and the use thereof for disrupting whole virus are known in the art. Preferred detergents for such use are alkali (lithium or sodium) dodecyl sulfate, sulfobetain, deoxylcholate and laurolylsarcosine (Sarcosyl).

In the case where the rubella virus antigen is to be supported on a solid support for use in an agglutination assay technique, the detergent or surfactant is one which is capable of disrupting and solubilizing the virus to provide soluble virus antigen having a molecular weight such that when supported on a particle, the sensitized particle remains mono-dispersed. In general, when using the rubella virus antigen for the sensitization of a particle, the soluble antigen does not have a molecular weight in excess of 125,000, and most generally not in excess of 100,000, as determined by acrylamide gel electrophoresis.

As hereinabove indicated, the surfactant is employed in an amount which is sufficient to disrupt and solubilize the virus and which does not destroy the antigenic characteristics thereof (too much detergent may destroy the antigenic characteristics). In general, the surfactant to virus weight ratio is an amount of from 0.2:1 to 5:1, preferably from 0.5:1 to 1:1. The selection of an optimum amount is deemed to be within the scope of those skilled in the art from the teachings herein.

The treatment of the purified virus is effected at a temperature which does not denature the virus proteins, with such temperature generally not exceeding about 30°C, with a temperature of from 20°C to 25°C being most convenient. Similarly, the pH is selected so as to maintain stability, with the pH being generally at 8.5, with the optimum pH generally being in the order of from 8.0 to 9.0.

The treatment of the purified virus with the surfactant is for a period of time sufficient to disrupt the virus and effect solubilization thereof. In general, such disruption and solubilization can be accomplished in time periods in the order of from 5 to 120 minutes, however, in some cases longer or shorter times may be applicable.

The selection of an optimum treatment time is deemed to be within the scope of those skilled in the art from the teachings herein.

Patentee has found that by using a surfactant to disrupt and solubilize the whole rubella virus, as hereinabove described, it is possible to provide soluble rubella virus antigen which retains its antigenicity.

A procedure for disruption and solubilization of whole virus, as hereinabove described, has been previously practiced in the art; for example, Vaheri et al. "Structural Proteins and Subunits of Rubella Virus", Journal of Virology, P. 10-16 (Jan. 1972). In addition, it is known that such a procedure is capable of recovering the structural proteins of the whole rubella virus, with there being three principal structural proteins, namely a structural protein with a molecular weight in the order of from 60,000 to 65,000 daltons, a structural protein with a molecular weight in the order of from 40,000 to 50,000 daltons, and a structural protein having a molecular weight in the order of from 32,000 to 38,000 daltons. The Patentee has also found evidence of a structural protein having a molecular weight of from 100,000 to 120,000 daltons.

The Patentee has found that the structural proteins recovered by such a procedure retain antigenic characteristics, and in addition, such structural proteins can be used in an assay for rubella antibody. Furthermore , the patentee has found that such structural proteins are capable of detecting early phase rubella antibody, i.e., the rubella antibody present in serum or plasma within ten days of onset of rubella rash. The term "rubella virus antigen" as used herein encompasses one or more of such structural proteins recovered by such procedure.

In accordance with an aspect of the present invention, the patentee has found that disruption and solubilization of whole rubella virus produces a soluble product which is antigenic and which is capable of reacting with rubella antibody, including the early phase antibody. Thus, by using a product prepared by such a procedure in an assay for rubella antibody; and in particular on a solid support, it is possible to detect rubella antibody even during the early phase.

As hereinafter described, the recovered product is of particular value for a direct agglutination assay, and applicant has found that such soluble rubella virus antigen may be supported on a latex particle (in particular a polystyrene) without the problem of self agglutination, i.e., the sensitized particles remain mono-dispersed.

The purified whole virus which is treated with surfacants is a virus which is produced in a tissue culture by procedures known in the art, and which is subsequently purified to remove non-virus lipids, nucleic acids, and non-viral proteins.

The tissue culture growth of rubella virus wherein rubella virus infected cells are raised in a suitable culture medium is well known in the art. The cells that are suitable for tissue culture growth to produce the rubella virus includes Vero cells, Baby Hamster Kidney, Procine Stabile Kidney or Serum Institute Rabbit Cornea. In general, tissue cultures conventionally used for producing rubella virus are also suitable for the purposes of the present invention.

The virus may then be purified by procedures known in the art; e.g. as disclosed by Baheri et al., supra. In accordance with a preferred embodiment, the virus is purified in accordance with a procedure of the present invention.

More particularly, the procedure for purifying virus in accordance with the invention, involves, treating concentrated virus with hydroxyl apatite gel in an aqueous solution of controlled ionic strength and pH.

More particularly, after filtration and concentration, the virus is contacted with hydroxyl apatite gel in an aqueous solution having an ionic strength which is great enough to minimize or prevent adsorption of the virus by the gel, and which is low enough to allow the non-virus proteins to be adsorbed by the gel. The ionic strength is maintained by the use of phosphate ions, with the phosphate ions being present at a molarity of from 0.05M to 1.5M to provide for effective adsorption of non-virus proteins and nucleic acids, without significant adsorption of the virus. The phosphate molarity in most cases is at least 0.08 M.

In addition, the adsorption is conducted at a pH in the order of from 6 to 9, most generally in the order of from 7 to 8. The pH of the solution is maintained by the use of a suitable buffer. The adsorption may be conducted in the presence of EDTA at a concentration from .01M to .0001M. EDTA as well as other chelating agents increases adsorption of non-viral proteins and nucleic acids, and aids in minimizing the adsorption of viral proteins.

By proceeding in accordance with the purification of the invention, the high molecular weight proteins and nucleic acids are adsorbed by the gel to thereby separate the virus protein from the non-virus proteins having similar molecular weights

After such adsorption, the lower molecular weight proteins still remaining in the fluid may be separated by conventional procedures. Thus, for example, further separation may be accomplished by centrifugation through a barrier layer or cushion as known in the art. In particular, the virus protein is centrifuged through a suitable barrier layer such as sucrose, glycerol, cesium chloride or cesium sulfate, with the lower molecular weight proteins remaining above the barrier, and the virus being centrifuged through the barrier, as a separate layer. The fluid containing the low molecular weight proteins and the barrier layer are then removed leaving a virus protein essentially free of non-virus proteins, nucleic acids or lipids. In general, the purified virus contains less than 1%, most generally less than 0.1% of non-virus lipids, nucleic acids and proteins.

It is to be understood that although the hereinabove described procedure for purifying the rubella virus is preferred, other procedures for separating non-virus proteins, lipids and nucleic acids can also be employed for purifying the rubella virus for subsequent treatment with surfactant to thereby produce the soluble rubella virus.

The viral antigen which is prepared by disruption and solubilization of whole virus may be supported on a solid support for use in an assay.

The rubella virus antigen prepared by disrupting and solubilizing whole rubella virus may then be supported on a solid support for use in an assay for rubella virus antibody. Such supported rubella virus antigen is capable of reacting with early phase rubella virus antibody. In accordance with the preferred embodiment, the rubella virus antigen is supported on a particulate support for use in an agglutination assay; however, it is to be understood that the rubella virus antigen may be supported on a non-particulate support (or for that matter on a particulate support) for use in an assay for rubella virus antibody by procedures other than the agglutination technique. Thus, for example, the supported rubella virus antigen may be supported on a solid support for use in an assay for rubella virus antibody by a radioimmunoassay, fluorescent or enzyme assay technique. Similarly, the rubella virus antigen of the present invention may be employed for the assay of rubella virus antibody in unsupported form by use of such techniques.

The antigen may be supported on any one of a wide variety of solid supports which are capable of supporting the antigen, and which can be used in the assay procedure without interfering with the immunochemical reaction. Moreover, the support should be one which is stable; i.e., not adversely affected by the prepared antigen. The antigen may be supported on the support by an adsorption technique, or by covalent coupling, either by activation of the support, or by the use of a suitable coupling agent, or by use of reactive groups on the support. Such procedures are generally known in the art.

The support may be any one of a wide variety of supports, and as representative examples of suitable supports there may be mentioned: synthetic polymer suppports, such as polystyrene, polypropylene, substituted polystyrene (e.g., aminated or carboxylated polystyrene), polyacrylamides, polyamides and polyvinylchloride ; glass beads and agarose. The supports may include reactive groups; e.g., carboxyl and amino groups to permit direct linking of the virus antigen to the support.

In accordance with preferred embodiment, the particulate support is either a polystyrene, aminated polystyrene, carboxylated polysytrene or a polyvinylchloride.

As hereinabove indicated, the antigen may be supported on the support by the use of an adsorption technique, or by covalent coupling with a coupling agent. As representative examples of suitable coupling agents there may be mentioned: dialdehydes; for example glutaraldehyde, succinaldehyde and malonaldehyde; unsatured aldehydes, e.g., acrolein, methacrolein and crotonaldehyde; carbodiimides; diisocyanates; dimethyladipimate; and cyanuric chloride. The selection of a suitable coupling agent should be apparent to those skilled in the art from the teachings herein.

Similarly, the antigen may be supported by activation of a suitable support; for example, cyanogen, bromide activated agarose.

In accordance with a preferred embodiment, as hereinabove noted, the soluble rubella virus antigen is supported on a particulate support which is either polystyrene (substituted or unsubstituted) or polyvinylchloride; most preferably polystyrene.

In some cases, the soluble antigen may be supported by an adsorption technique, in other cases, it may be necessary to employ covalent coupling.

The virus antigen sensitized particulate support is preferably prepared for use in an assay in which rubella virus antibody is determined by an agglutination technique. The particulate support is provided with an effective amount of the antigen for the assay, while preventing excessive amounts which may result in bridging of the antibody to a single particle. In general the weight ratio of soluble rubella antigen to support is from 1:100 to 1:5000. The selection of an optimum amount is deemed to be within the scope of those skilled in the art from the teachings herein.

In accordance with one technique, after the antigen is adsorbed on the particles, the support, including the adsorbed antigen, is further coated with protein which does not adversely affect the subsequent immunochemical reaction in order to provide a protein coating on the portion of the support which does not including the antigen. As should be apparent, the protein coating should not immunologically react with either the rubella virus antigen or with sera to be used in the assay. As examples of suitable proteins there may be mentioned: bovine serum albumin or ovalbumin. The selection of a suitable protein to saturate the spaces between the rubella virus antigen on the support is deemed to be within the scope of those skilled in the art from the teachings herein.

It is to be understood that such coating with protein is not required for producing sensitized particles for use in an agglutination assay.

After the rubella virus antigen has been supported on a solid support, as generally practiced in the art for the production of sensitized particles for use in an agglutination assay, the sensitized particles are treated with a liquid containing polyoxyethylene sorbitan monolaurate (Tween® 20) at a weight ratio to the polystyrene of 0.1:1 to 10.

The sensitized particles are preferably a synthetic polymer and in particular a polystyrene [substituted (carboxylated or aminated) or unsubstituted] or polyvinylchloride latex. Patentee has found that sensitization of such particles with soluble rubella virus antigen prepared, as hereinabove described, produces a sensitized particles which remains mono-dispersed (no self agglutination), whereby such sensitized latex particles may be effectively employed in a direct agglutination assay for rubella antibody. Such sensitized particles are capable of detecting early phase rubella antibody. In addition, such sensitized particles are capable of providing a direct agglutination assay having a high sensitivity for rubella antibody.

The rubella virus antigen sensitized particle prepared in accordance with the invention are suitable for use in a kit and assay for rubella virus antibody by a direct agglutination procedure. Such kit may include, in addition to the sensitized rubella virus particles, as hereinabove described, in a suitable container therefor, a reactive serum control (contains rubella antibody) and a non-reactive serum control (no rubella antibody) in suitable containers therefor. In accordance with a preferred embodiment, in addition to the reagents, there is provided a test card on which the assay is effected. The test card has a flat testing surface which include suitably marked areas (for example, a test circle) for placing one or more samples to be assayed, as well as suitably marked areas for each of the serum controls. The test card and reagents may be included in a single kit package.

In the agglutination assay, undiluted serum or diluted serum (e.g. 1:10) is contacted with the sensitized particles followed by mixing, with the presence of the antibody against rubella virus being evidenced by visible agglutination.

Such rubella virus antigen sensitized particles may also be employed in a quantitative assay for rubella virus antibody.

In a quantitative assay, the sample to be assayed is serially diluted, as appropriate, and to each serial dilution there is added the particles sensitized with the soluble rubella antigen. The quantity of antibody in the sample is determined from the highest dilution giving any agglutination of the sensitized particles.

The quantitative or qualitative assay for rubella antibody may be effected on a card surface wherein the surface includes suitably marked areas for placing the sample and control to which the sensitized particles are added.

The invention will be further described with respect to the following examples:

EXAMPLE I

A. Production and Purification of Rubella Virus.

Confluent roller cultures (680 cm⁻) of Vero cells (a continuous culture line of cells derived from African Green monkey kidney) were innoculated with approximately 0.01 PFU of rubella virus per cell and maintained in a standard culture medium (Medium 199) containing .025 M hepes buffer. pH 7.4, and 2% (vol.vol) of the filtrate obtained by forcing fetal bovine serum through a membrane designed to retain molecules of 100.000 molecular weight and greater (Amicon® XM-100 membrane). The medium was changed daily. and the culture fluids having a hemagglutination titer greater than 16 were made to contain 0.01 M Tris base and 0.01 M EDTA. After incubation at 4°C for 1 hour, they were concentrated in an Amicon hollow fiber dialyzer-concentrator to 1 10 the original volume. After clarification at 5.000 x g for 20 minutes, the pH was adjusted to 7.6 at 22°C and 1 10 volume of hydroxylapatite suspension was added, and the slurry was incubated at 4°C with mixing, overnight. The hydroxylapatite was removed by centriguation at 5.000 x g for 15 minutes, after which 30 ml of the concentrate was layered over 9 ml of 69% (wt wt) glycerol in a Beckman SW28 tube. The virus was sedimented at 82,000 x g for 16 hours at 4°C, and the resultant pellet was resuspended in 0.01 M carbonate buffer, pH 9.5 (coating buffer). The purified virus was assayed for hemagglutinin content and stored at -70°C.

B. Solubilization of Purified Virus.

The purified virus in 0.01 M carbonate buffer. pH 9.5, was solubilized by treatment with sodium dodecyl sulfate (SDS). The purified virus was made to contain 0.05% (w v) SDS and was incubated for 30 minutes at room temperature.

C. Preparation of Sensitized Latex.

Commercial suspensions of polystyrene latex (0.9 micron diameter particles) were washed four times with 25 volumes each of the coating buffer and were resuspended in the coating buffer to provide 3% solids ($^{vol}$/vol.). The latex suspension was added directly to the solubilized virus at a ratio of 2 volumes of the 3% latex to 1 volume of solubilized virus and the suspension was mixed by tumbling for 16 hours at room temperature. The sensitized latex was washed twice with 20 volumes of 1% bovine serum albumin in phosphate buffered saline (BSA-PBS) and resuspended at 0.5% in 1% BSA-PBS contained 0.05% polyoxyethylene sorbitan monolaurate surface active agent (Tween 20) and 0.02% gentamiacin.

D. Latex Agglutination Test for Rubella Virus Antibodies.

Glass plates with 1.4 cm fused circles were employed. Serial 2-fold dilutions of serum were prepared in 1% BSA-PBS-Tween 20 and 25 ul of each dilution was placed in separate wells. After adding 25 ul of sensitized latex, the serum and latex suspension was mixed and rotated 100 rpm for 5 minutes. The presence of antibody against rubella virus was evidenced by visible agglutination.

EXAMPLE II

Purified virus prepared in accordance with Example I was treated with a 1% aqueous solution of sarcosyl for 30 minutes at room temperature in coating buffer to disrupt and solubilize the virus.

The pH of the solubilized virus was adjusted to 6.5 with hydrochloric acid and mixed with two volumes of 3% carboxylated polystyrene latex (in phosphate buffer, pH 6.5) for 1 hour at 4° C.

To the solution was added 10 mg of a carbodiimide coupling agent and the mixture was mixed overnight at 4° C.

After centrifugation, the solids were resuspened in phosphate buffered saline (PBS) followed by centriguation and resuspension in PBS containing 1% BSA and 0.05% Tween 20.

The procedure covalently bound the soluble rubella virus antigen to the latex.

Provision of a testcard

In accordance with a preferred procedure, there is provided a test card for rubella antibody. The test card includes a marked circle for a reactive control, a marked circle for non-reactive control, as well as one or more test sample circles.

25 ul of undiluted serum sample is placed in an appropriately marked sample circle, and 25 ul of the reactive and non-reactive controls are placed in their respective circles.

With a micropipettor, there is added sensitized latex of Example III (approximately 15 ul), followed by rotation on a rotator (about 8 minutes), and gentle hand rotation.

The card is read microscopically in the wet state under a high intensity incandescent lamp.

The reactive control should show definite agglutination and the non-reactive control should show no agglutination.

Any serum samples showing any agglutination should be reported as reactive.

Numerous modifications and variations of the present invention are possible in light of the above teachings and, therefore, within the scope of the appended claims.

## Claims

1. A process for producing a solid support for an immunological assay the support being sensitized with soluble rubella virus antigen, comprising:
   deriving soluble rubella virus antigen having at least one structural protein of the virus and being immunoreactive with early phase rubella virus antibody by disruption of whole rubella virus with a surfactant or detergent for a time and in an amount sufficient to disrupt the virus without destroying its antigenic characteristics and
   supporting the solubilized antigen on a solid support.

2. The process of Claim 1 wherein the whole rubella virus is solubilized with a detergent in a ratio of detergent to virus of 0.2 : 1 to 5 : 1 for time of from 5 to 120 minutes.

3. The process of Claim 1 wherein the whole rubella virus is disrupted with a detergent in a detergent to virus ratio of 0.5 : 1 to 1 : 1.

4. The process of Claim 1 further comprising contacting the whole rubella virus with hydroxyl apatite in the presence of phosphate ion and at a pH of from 6 to 9, the phoshpate ion being present in a molarity of from 0.05 to 1.5 M to provide for adsorption of non-viral proteins without significant adsorption of viral protein.

5. The process of Claims 1 to 4 wherein the solubilized antigen is coated on synthetic polymer particles.

6. The process of Claim 5 wherein the synthetic polymers are selected from poyvinylchloride, polystyrene, aminated polystyrene and carboxylated polystyrene.

**Revendications**

1. Procédé de production d'un support solide pour un essai immunologique , le support étant sensibilisé avec un antigène soluble du virus de la rubéole , comprenant :
l'extraction d'un antigène soluble du virus de la rubéole ayant au moins une protéine structurale du virus et étant immunoréactif avec un anticorps du virus de la rubéole à un stade précoce par la lyse de l'ensemble du virus de la rubéole avec un tensio-actif ou détergent pendant un temps suffisant et avec une quantité suffisante pour lyser le virus sans détruire ses caractéristiques antigéniques et
l'application de l'antigène solubilisé sur un support solide .

2. Le procédé de la revendication 1 où l'ensemble du virus de la rubéole est solubilisé avec un détergent dans un rapport détergent sur virus de 0,2/1 à 5/1 pendant une durée allant de 5 à 120 minutes.

3. Le procédé de la revendication 1 où l'ensemble du virus de la rubéole est lysé avec un détergent dans un rapport détergent sur virus de 0,5/1 à 1/1.

4. Le procédé de la revendication 1 comprenant de plus la mise en contact de l'ensemble du virus de la rubéole avec de l'hydroxylapatite en la présence d'ion phosphate et à un pH de 6 à 9, l'ion phosphate étant présent avec une molarité de 0,05 à 1,5 M pour fournir une adsorption des protéines non-virales sans adsorption significative de protéines virales.

5. Le procédé des revendications 1 à 4 où l'antigène solubilisé recouvre des particules de polymères synthétiques.

6. Le procédé de la revendication 5 où les polymères synthétiques sont choisis parmi le poly-(chlorure de vinyle), le polystyrène , le polystyrène aminé et le polystyrène carboxylé.

**Ansprüche**

1. Verfahren zur Herstellung eines festen Trägers für eine immunologische Analyse, wobei der Träger mit löslichem Rubella-Virus-Antigen solubilisiert ist, umfassend
das Gewinnen von löslichem Rubella-Virus-Antigen, das wenigstens ein Strukturprotein des Virus aufweist und gegenüber einer frühen Phase des Rubella-Virus-Antikörpers immunreaktionsfähig ist, durch Aufbrechen des ganzen Rubella-Virus mittels eines Tensids oder Waschmittels für die Dauer einer Zeitspanne und in einer Menge, die ausreichen. um das Virus aufzubrechen, ohne seine charakteristischen antigenen Eigenschaften zu zerstören, und
das Aufziehen des solubilisierten Antigens auf einen festen Träger.

2. Verfahren nach Anspruch 1, worin das ganze Rubella-Virus mit einem Waschmittel in einem Verhältnis Waschmittel zu Virus von 0.2 1 bis 5 : 1 über eine Zeitspanne von 5 bis 120 min solubilisiert wird.

3. Verfahren nach Anspruch 1. worin das ganze Rubella-Virus mit einem Waschmittel in einem Verhältnis Waschmittel zu Virus von 0.5 . 1 bis 1 : 1 aufgebrochen wird.

4. Verfahren nach Anspruch 1. weiterhin umfassend das In-Berührung-Bringen des ganzen Rubella-Virus mit Hydroxylapatit in Gegenwart von Phosphat-Ionen und bei einem pH-Wert von 6 bis 9, wobei das Phosphat-Ion in einer Molarität von 0.05 bis 1.5M vorliegt, um eine Adsorption nicht-viraler Proteine ohne nennenswerte Adsorption viraler Proteine zu erreichen.

5. Verfahren nach den Ansprüchen 1 bis 4. worin das solubilisierte Antigen als Schicht auf synthetische Polymer-Teilchen aufgezogen wird.

6. Verfahren nach Anspruch 5. worin die synthetischen Polymeren aus Polyvinylchlorid. Polystyrol, aminiertem Polystyrol und carboxyliertem Polystyrol ausgewählt sind